# EUROPEAN PATENT SPECIFICATION

(11) **EP 0 639 372 B1**
(45) Date of publication and mention of the grant of the patent: **13.11.2002**
(21) Application number: 94401864.7
(22) Date of filing: 17.08.1994
(51) Int. Cl.: A61K 9/12, A61K 7/48, C09K 3/30, A61K 7/00

(54) **Device for distributing a therapeutic or cosmetic substance, where the inert carrier is hexamethyldisiloxane, and composition for use in this device**
Vorrichtung zur Verteilung einer therapeutischen oder kosmetischen Substanz, wobei der inerte Träger Hexamethyldisiloxan ist und Zusammensetzung für den Gebrauch in dieser Vorrichtung
Dispositif de distribution d'une substance thérapeutique ou cosmétique dont le véhicule inerte est l'hexaméthyldisiloxane, et composition destinée à être utilisée dans le dispositif

(30) Priority: 18.08.1993 FR 9310075
(43) Date of publication of application: 22.02.1995
(73) Proprietor: Dow Corning France, 69432 Lyon Cedex 3 (FR)
(72) Inventor: Appino, James, Doylestown PA 18901 (US); Aiache, Jean-Marc, F-6300 Clermont-Ferrand (FR)
(74) Representative: Ahner, Francis

(56) References cited:
- EP-A- 0 229 640
- EP-A- 0 468 564
- EP-A- 0 478 456
- DE-A- 3 330 447
- DE-A- 3 810 730
- SEIFEN, OLE, FETTE, WACHSE, vol.113, no.18, Novembre 1987, AUGSBURG DE pages 685 - 689 J. ROIDL 'Flüchtige Trägersubstanzen für Wirkstoffe'

## Description

La présente invention concerne un dispositif permettant de distribuer sous forme de particules d'aérosol une substance thérapeutique ou cosmétique;

Elle concerne également une composition pulvérisable destinée notamment à être utilisée dans le dispositif selon l'Invention et un procédé de traitement cosmétique.

Les bombes aérosols permettant de distribuer diverses substances, notamment à usage cosmétique ou thérapeutique, et qui comprennent des gaz fluorocarbonés (CFC ou HFA) comme gaz propulseurs, sont progressivement interdites compte tenu des effets néfastes desdits gaz sur l'atmosphère.

Afin de remplacer les gaz propulseurs fluorocarbonés, on a proposé différents dispositifs faisant intervenir d'une part un gaz propulseur (non fluorocarboné) et d'autre part, un véhicule inerte permettant d'accompagner la substance active jusqu'à son lieu d'application.

Certains de ces dispositifs appelés communément bi-poches sont formés d'un récipient extérieur rigide et d'un récipient intérieur élastique dont le bord supérieur est fixé au récipient extérieur. Le récipient extérieur est surmonté d'un couvercle porte-valve posé sur le bord supérieur du récipient extérieur et d'une valve de débit qui s'étend dans le récipient intérieur, un gaz propulseur étant placé dans l'espace libre séparant le récipient extérieur du récipient intérieur.

Lorsque la valve de débit est actionnée, le gaz propulseur refoule le produit contenu dans le récipient intérieur vers l'extérieur par la valve de débit qui est alors ouverte.

Ces dispositifs sont notamment décrits dans les demandes de brevet FR-A-2 436 085 ou FR-A-2 663 909.

D'autres dispositifs sont constitués d'un récipient rigide surmonté d'un corps de pompe comprenant une chambre de pompe à volume variable, à l'intérieur de laquelle est logé un piston, avec un trou d'entrée pour établir une communication de remplissage de la chambre avec le récipient et un trou de sortie pour permettre l'émission à l'extérieur de la substance active. Dans ce cas, le gaz propulseur est fourni par la compression d'un fluide, qui est le plus souvent de l'air, à l'intérieur du récipient au moyen de l'actionnement du piston de la chambre de pompe.

Ces dispositifs sont notamment décrits dans les demandes de brevet FR-A-2 647 418 et FR-A-2 643 338.

Néanmoins les véhicules inertes contenus dans les récipients en mélange avec la substance active présentent certains inconvénients liés à leur nature chimique propre ce qui rend le fonctionnement de tels dispositifs peu satisfaisants.

C'est le cas notamment des véhicules inertes à base d'alcool éthylique.

On connaît par ailleurs l'utilisation en cosmétique ou en médecine pour des applications topiques de solutions, gels ou bâtonnets comportant comme substance favorisant l'étalement, des polydiorganosiloxanes.

Le brevet US 4 346 079 propose une composition anti-transpirante contenant une solution alcoolique gélifiée d'un composé anti-transpirant, l'agent gélifiant comprenants des sels d'acide gras tels que le stéarate de sodium et une faible quantité d'un siloxane dont la viscosité est comprise entre 0,01 à 25 centistokes comme les polydiméthylsiloxanes, les polyphénylméthylsiloxanes et les polydiméthylsiloxanes cycliques.

Par ailleurs, la demande de brevet EP-A-0 211 555 décrit une composition silicone transparente comportant une faible quantité d'un alcool gras liquide ou d'un alcool polyoxyalcoylé liquide. Parmi les silicones utilisés, le document cite les polyméthylsiloxanes linéaires ou cycliques ayant un à six méthylsiloxanes. Ces compositions trouvent une application en cosmétique (shampooings, déodorants, anti-transpirants, lotions et parfums) En d'autres termes, les silicones volatils dans ces réalisations sont des véhicules assurant un bon étalement de la substance active et/ou une bonne pénétration de celle-ci lorsque la composition a déjà été déposée sur le support à traiter.

Le document DE 38 10730 décrit une composition pulvérisable cosmétique qui comprend une quantité d'hexaméthyldisiloxane qui ne peut donner de résultats satisfaisants dans le cadre d'un dispositif de distribution sous forme de particules d'aérosol d'une substance thérapeutique ou cosmétique.

Le document EP-A-468 564 décrit une composition cosmétique dans laquelle à l'exemple 1 le silicone volatil est le Dow Corning 345 (pentamer).

Le document DE 33 30 447 indique de manière très générale l'utilisation de polymère de diméthylsiloxane pour les aérosols (page 7, lignes 13 à 16).

Le document EP 478 456 décrit une composition antifongique sous forme de spray sec incorporant divers silicones.

De façon inattendue, on a trouvé que l'hexaméthyldisiloxane de volatilité suffisante pouvaient être utilisés en tant que véhicule inerte des dispositifs de distribution sous forme d'aérosol d'une substance active thérapeutique ou cosmétique.

L'objet de la présente invention est donc de proposer un dispositif comportant un tel véhicule inerte et permettant une application tout à fait satisfaisante de la substance active sur la peau.

Selon l'invention, le terme de véhicule inerte est employé pour désigner le milieu de dispersion qui amènera la substance active de l'intérieur du réservoir au support à traiter.

Il s'agit donc d'une fonction disjointe de celle consistant à faciliter l'étalement sur la peau d'une composition qui y a déjà été déposée par un moyen approprié.

Par ailleurs, on a constaté de façon inattendue que le dépôt ainsi obtenu par l'utilisation du dispositif présentait une grande homogénéité et une grande régularité, tout en procurant une sensation agréable comparée aux dépôts similaires effectués par d'autres moyens.

L'invention est donc relative à un dispositif, permettant de distribuer sous forme d'aérosol une substance active thérapeutique ou cosmétique, constitué d'un récipient contenant une composition comportant la substance active et un véhicule inerte, d'une valve de débit s'étendant dans ledit récipient et d'un moyen de propulsion caractérisé en ce que la composition consiste, en pourcentage en poids, en:
- 75 à 95 % d'hexaméthyldisiloxane.
- 0,01 à 15 % de substance thérapeutique ou cosmétique,
- 0 à 10 % d'agent améliorant la thixotropie, de préférence au moins 1%,
- 0 à 20 % de charges de densitification ou additifs cosmétiques.

Les dispositifs qui sont utilisés dans le cadre de la présente invention peuvent comporter d'une part un gaz propulseur et d'autre part un véhicule inerte. Ces dispositifs peuvent être du type bi-poche ou à chambre de pompe à volume variable.

De façon connue, les dispositifs bi-poches sont formés d'un récipient interne élastique contenant la composition et d'un récipient externe rigide, l'espace libre séparant le récipient externe du récipient interne étant occupé par un gaz propulseur. Les dispositifs à chambre de pompe à volume variable sont formés d'un récipient rigide contenant la composition, associé à une chambre de pompe communicant avec ledit récipient.

Ils peuvent être également du type dont le piston de la pompe qui est dans la chambre, actionné par le poussoir, permet l'expulsion de la composition sans nécessiter l'action de gaz propulsifs. Ces dispositifs sont particulièrement préférés et un exemple d'un tel dispositif est celui commercialisé par la société Valois Pharm. (France), sous la référence VP3.

L'hexaméthyldisiloxane est fabriqué par exemple par la société DOW-CORNING et porte la référence Q7-9179 ou par RHONE POULENC référence Silbione Huile 70041 V 0,65, Les propriétés physico-chimiques de ce produit sont notamment décrites dans les brochures commerciales éditées par ces sociétés. Ces composés sont par ailleurs connus pour leur compatibilité avec une utilisation en cosmétique ou en dermatologie sous forme topique.

De préférence, ces composés présentent une pureté supérieure ou égale, de préférence supérieure, à 99 % ou avantageusement 99,2 % et passent au tamis de 400 mesh.

Le véhicule inerte et la substance active forment un mélange thixotrope, c'est-à-dire qu'il est au repos sous forme de gel alors que l'application d'un mouvement de haut en bas, notamment, permet de le retransformer en liquide prêt à être expulsé sous l'action du gaz propulseur.

Afin d'améliorer le caractère thixotrope du mélange, il est avantageux d'ajouter à la composition comportant le véhicule inerte un agent connu pour améliorer la thixotropie. Cet agent sera notamment à base de silice comme le composé connu sous le nom de marque Aérosil® (commercialisé par la société DEGUSSA).

Par ailleurs la composition comprendra avantageusement des charges de densification, notamment à base de silice comme le talc et des additifs améliorant la présentation notamment olfactive du mélange comme des parfums.

La composition sera soit sous forme de solution lorsque la substance active est soluble dans le véhicule inerte, soit sous forme de suspension en cas d'insolubilité de la substance active dans le véhicule inerte.

Ainsi, la substance active transportée sous forme dispersée par le véhicule peut être soit sous forme liquide, soit sous forme solide.

De préférence, la substance active sera transportée sous forme de poudre et en conséquence, les composés formant la substance active seront choisis parmi ceux qui forment une suspension dans le véhicule inerte.

Parmi ces composés, il est avantageux d'utiliser comme substance active un antifongique comme les imidazoles ou les triazoles, notamment à base d'éconazole. Bien entendu, d'autres antifongiques peuvent également être utilisés dans le cadre de la présente invention.

Comme autres substances actives pouvant être utilisées avantageusement dans le cadre de l'invention, on peut citer notamment les produits cosmétiques suivants: "Déodorants, antiperspirants, antiseptiques cutanés et les produits pharmaceutiques choisis parmi les hémostatiques, les antibiotiques, les polymères protecteurs, cicatrisants, les hormones, les substances absorbées par la peau capables d'une activité générale (cardiotonique par ex.)".

De préférence, la composition pulvérisable consiste en pourcentage en poids en:
- 80 à 90 % d'hexaméthyldisiloxane,
- 0,1 à 5 % de substance thérapeutique ou cosmétique,
- 0 à 10 % d'agent améliorant la thixotropie de préférence au moins 1%,
- 0 à 20 % de charges ou additifs, de préférence au moins 5 %.

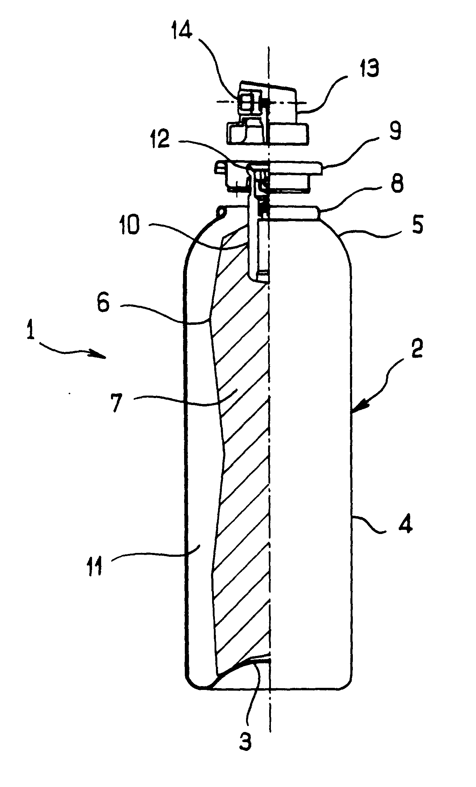

A titre d'illustration de la présente invention la figure unique ci-jointe illustre un dispositif bi-poche tel qu'il est connu dans l'art, pouvant convenir à la mise en oeuvre de la présente invention.

La figure représente une demie coupe selon l'axe longitudinal d'un tels dispositif.

Conformément à cette figure, le dispositif distributeur 1 comporte un corps de récipient externe rigide 2 constitué d'un fond 3 à partir duquel s'étend verticalement vers le haut une paroi latérale périphérique 4 terminée à son extrémité supérieure par une paroi convergente 5. Un récipient intérieur en matière élastique souple 6 contenant la composition 7 de substance active et de véhicule inerte est logé à l'intérieur du corps de récipient, le bord du récipient intérieur étant fixé au récipient extérieur et un couvercle porte-valve 8 étant posé sur le bord supérieur du récipient extérieur et étant surmonté d'une valve de débit 9 qui s'étend dans le récipient extérieur par un embout coudé 10.

Un gaz propulseur 11 est placé dans l'espace libre séparant le corps de récipient extérieur du récipient intérieur.

L'embout coudé 10 à son extrémité supérieure est relié à la buse d'admission 12 puis à l'ouverture 14 du dispositif de distribution par l'actionnement du bouton poussoir 13.

L'invention peut également être réalisée à l'aide de dispositifs comprenant une chambre de pompe à volume variable comme décrit par exemple dans les demandes de brevet FR-A-2 436 (0)85 et 2 663 909 ou tels qu'ils sont bien connus de l'homme du métier.

L'invention a également pour objet une composition pulvérisable destinée notamment à être utilisée dans un dispositif tel qu'il vient d'être décrit précédemment et consistant en pourcentage en poids en :
- 75 à 95 % de d'hexaméthyldisiloxane,
- 0,01 à 15 % de substance thérapeutique ou cosmétique,
- 0 à 10 % d'agent améliorant la thixotropie de préférence au moins 1 %,
- 0 à 20 % de charges ou additifs.

L'invention a également pour objet l'utilisation de l'hexaméthyldisiloxane comme véhicule inerte dans les dispositifs de distribution de particules d'aérosol selon l'invention.

L'invention a également pour objet un procédé de traitement cosmétique de la peau, caractérisé en ce qu'on applique à un endroit de celle-ci une dose efficace d'une substance active à l'aide d'un dispositif tel que décrit précédemment.

Selon une variante, la substance appliquée est à usage cosmétique et est notamment sous la forme d'une poudre.

De façon inattendue, la couche ainsi déposée présente une uniformité et une homogénéité remarquable qu'il n'est pas possible d'atteindre avec les autres véhicules inertes.

Par ailleurs, si l'on utilise des véhicules inertes à base de composés fluorocarbonés tels que les HFA ou les CFC, la matière pulvérisée se présente sous la forme d'un brouillard dont l'application ne peut être dirigée de façon aussi efficace que lorsque l'on utilise le véhicule inerte selon l'invention.

L'invention est maintenant illustrée par un exemple de réalisation donné à titre illustratif.

La composition suivante est utilisée :
- nitrate d'éconazole 0,2 g,
- talc 2 g
- Aérosil 200 (silice colloïdale ) 0,6 g
- hexaméthyldisiloxane 17,16 g commercialisé par la société Dow Corning sous la référence Q7-9179,
- parfum 0,04 g

Le propulseur occupant l'espace libre entre les deux récipients est de l'azote. La pression exercée sur la poche (réservoir souple) est de 8 bars.

La mise sous pression peut également être exercée par une pompe mécanique.

## Claims

1. Device enabling a therapeutic or cosmetic active substance to be dispensed in the form of aerosol particles, consisting of a container (6) containing a composition (7) including the active substance and an inert vehicle, a flow valve (9) extending into the said container and a means of propulsion (11), **characterized in that** the composition consists, in percentage by weight, of:
- 75 to 95% of hexamethyldisiloxane,
- 0.01 to 15% of therapeutic or cosmetic substance,
- 0 to 10% of agent improving the thixotropy, preferably at least 1%, and
- 0 to 20% of cosmetic additives or densifying fillers.

2. Device according to Claim 1, **characterized in that** the composition comprises an agent based on silica which improves the thixotropy of the said vehicle.

3. Device according to Claim 1, **characterized in that** the active substance is in powder form.

4. Device according to one of Claims 1 to 3, **characterized in that** the active substance is an antifungal agent, in particular one which is based on econazole.

5. Device according to one of the preceding claims, **characterized in that** it is of the two-compartment type.

6. Device according to one of Claims 1 to 4, **characterized in that** it is of the variable-volume pump chamber type.

7. Cosmetic or pharmaceutical sprayable composition consisting in percentage by weight of:
- 75 to 95% of hexamethyldisiloxane,
- 0.01 to 15% of therapeutic or cosmetic substance,
- 0 to 10% of agent improving the thixotropy, preferably at least 1%, and
- 0 to 20% of fillers or additives, preferably at least 5%.

8. Sprayable composition according to Claim 7, consisting in percentage by weight of:
- 80 to 90% of hexamethyldisiloxane,
- 0.1 to 5% of therapeutic or cosmetic substance,
- 0 to 10% of agent improving the thixotropy, preferably at least 1%, and
- 0 to 20% of fillers or additives, preferably at least 5%.

9. Cosmetic treatment process for the skin, **characterized in that** an effective dose of an active substance is applied to an area of the skin using a device according to one of Claims 1 to 6.

10. Use of hexamethyldisiloxane as an inert vehicle in the manufacture of a cosmetic or pharmaceutical product intended to be presented in the form of an aerosol particle distributor, **characterized in that** the product composition consists, in percentage by weight, of:
- 75 to 95% of hexamethyldisiloxane,
- 0.01 to 15% of therapeutic or cosmetic substance,
- 0 to 10% of agent improving the thixotropy, preferably at least 1%,
- 0 to 20% of cosmetic additives or densifying fillers.

11. Use of hexamethyldisiloxane according to Claim 10, **characterized in that** the composition comprises an agent based on silica which improves the thixotropy of the vehicle.

12. Use of hexamethyldisiloxane according to either of Claims 10 and 11, **characterized in that** the active substance is in powder form.

13. Use of hexamethyldisiloxane according to one of Claims 10 to 12, **characterized in that** the active substance is an antifungal agent, in particular one which is based on econazole.

14. Use of hexamethyldisiloxane according to one of Claims 10 to 13, **characterized in that** the aerosol particle distributor is of two-compartment type.

15. Use of hexamethyldisiloxane according to one of Claims 10 to 13, **characterized in that** the aerosol particle distributor is of the variable-volume pump chamber type.

## Revendications

1. Dispositif permettant de distribuer sous forme de particules d'aérosol une substance active thérapeutique ou cosmétique constitué d'un récipient (6) contenant une composition (7) comportant la substance active et un véhicule inerte, d'une valve de débit (9) s'étendant dans ledit récipient et d'un moyen de propulsion (11), **caractérisé en ce que** la composition consiste, en pourcentage en poids, en:
- 75 à 95 % d'hexaméthyldisiloxane,
- 0,01 à 15 % de substance thérapeutique ou cosmétique,
- 0 à 10 % d'agent améliorant la thixotropie, de préférence au moins 1 %,
- 0 à 20 % de charges de densitification ou additifs cosmétiques.

2. Dispositif selon la revendication 1, **caractérisé en ce que** la composition comprend un agent à base de silice améliorant la thixotropie dudit véhicule.

3. Dispositif selon la revendication 1, **caractérisé en ce que** la substance active est sous forme de poudre.

4. Dispositif selon l'une des revendications 1 à 3, **caractérisé en ce que** la substance active est un antifongique, notamment à base d'éconazole.

5. Dispositif selon l'une des revendications précédentes **caractérisé en ce qu'**il est du type bi-proche.

6. Dispositif selon l'une des revendications 1 à 4, **caractérisé en ce qu'**il est du type à chambre de pompe à volume variable.

7. Composition pulvérisable cosmétique ou pharmaceutique consistant en pourcentage en poids, en:
- 75 à 95 % d'hexaméthyldisiloxane,
- 0,01 à 15 % de substance thérapeutique ou cosmétique,
- 0 à 10 % d'agent améliorant la thixotropie, de préférence au moins 1 %,
- 0 à 20 % de charges ou additifs, de préférence au moins 5 %.

8. Composition pulvérisable selon la revendication 7, consistant en pourcentage en poids, en :
- 80 à 90 % d'hexaméthyldisiloxane,
- 0,1 à 5 % de substance thérapeutique ou cosmétique,
- 0 à 10 % d'agent améliorant la thixotropie de préférence au moins 1 %,
- 0 à 20 % de charges ou additifs, de préférence au moins 5 %.

9. Procédé de traitement cosmétique de la peau **caractérisé en ce qu'**on applique à un endroit de celle-ci une dose efficace d'une substance active au moyen d'un dispositif selon l'une des revendications 1 à 6.

10. Utilisation d'hexaméthyldisiloxane comme véhicule inerte dans la fabrication d'un produit cosmétique ou pharmaceutique destiné à être présenté sous la forme d'un distributeur de particules d'aérosol, **caractérisée en ce que** la composition du produit consiste en pourcentage en poids, en:
- 75 à 95 % d'hexaméthyldisiloxane,
- 0,01 à 15 % de substance thérapeutique ou cosmétique,
- 0 à 10 % d'agent améliorant la thixotropie, de préférence au moins 1 %,
- 0 à 20 % de charges de densitification ou additifs cosmétiques.

11. Utilisation d'hexaméthyldisiloxane selon la revendication 10, **caractérisée en ce que** la composition comprend un agent à base de silice améliorant la thixotropie du véhicule.

12. Utilisation d'hexaméthyldisiloxane selon l'une des revendications 10 et 11, **caractérisée en ce que** la substance active est sous forme de poudre.

13. Utilisation d'hexaméthyldisiloxane selon l'une des revendications 10 à 12, **caractérisée en ce que** la substance active est un antifongique, notamment à base d'éconazole.

14. Utilisation d'hexaméthyldisiloxane selon l'une des revendications 10 à 13, **caractérisée en ce que** le distributeur de particules d'aérosol est du type bi-poche.

15. Utilisation d'hexaméthyldisiloxane selon l'une des revendications 10 à 13, **caractérisée en ce que** le distributeur de particules d'aérosol est du type à chambre de pompe à volume variable.

## Patentansprüche

1. Vorrichtung, die es gestattet, eine aktive therapeutische oder kosmetische Substanz in Form von Aerosol-Partikeln zu verteilen, und aus einem Behälter (6), der eine Zusammensetzung (7) enthält, welche die aktive Substanz und einen inerten Träger umfasst, einem Ausstoßventil (9), das sich in den Behälter erstreckt, und aus einem Mittel zum Treiben (11) aufgebaut ist, **dadurch gekennzeichnet, dass** die Zusammensetzung in Gewichtsprozent besteht aus:
- 75 - 95 % Hexamethyldisiloxan
- 0,01 bis 15 % therapeutischer oder kosmetischer Substanz,
- 0 bis 10 % eines Mittels, das die Thixotropie verbessert, vorzugsweise mindestens 1 %,
- 0 bis 20 % Verdichtungs-Füllmitteln oder kosmetischen Zusätzen.

2. Vorrichtung nach Anspruch 1, **dadurch gekennzeichnet, dass** die Zusammensetzung ein Mittel auf der Basis von Siliciumdioxid umfasst, das die Thixotropie des Trägers verbessert.

3. Vorrichtung nach Anspruch 1, **dadurch gekennzeichnet, dass** die aktive Substanz in Form von Pulver vorliegt.

4. Vorrichtung nach einem der Ansprüche 1 bis 3, **dadurch gekennzeichnet, dass** die aktive Substanz ein Antifungusmittel ist, insbesondere auf der Basis von Econazol.

5. Vorrichtung nach einem der vorangehenden Ansprüche, **dadurch gekennzeichnet, dass** sie von der Zwei-Taschen-Art ist.

6. Vorrichtung nach einem der Ansprüche 1 bis 4, **dadurch gekennzeichnet, dass** sie von der Art mit einer Pumpenkammer mit variablem Volumen ist.

7. Zerstäubbare kosmetische oder pharmazeutische Zusammensetzung, die in Gewichtsprozent besteht aus:
- 75 bis 95 % Hexamethyldisiloxan,
- 0,01 bis 15 % therapeutischer oder kosmetischer Substanz,
- 0 bis 10 % eines Mittels, das die Thixotropie verbessert, vorzugsweise mindestens 1 %,
- 0 bis 20 % Füllstoffen oder Zusätzen, vorzugsweise mindestens 5 %.

8. Zerstäubbare Zusammensetzung nach Anspruch 7, die in Gewichtsprozent besteht aus:
- 80 bis 90 % Hexamethyldisiloxan,
- 0,1 bis 5 % therapeutischer oder kosmetischer Substanz,
- 0 bis 10 % eines Mittels, das die Thixotropie verbessert, vorzugsweise mindestens 1 %,
- 0 bis 20 % Füllstoffen oder Zusätzen, vorzugsweise mindestens 5 %.

9. Verfahren zur kosmetischen Behandlung der Haut, **dadurch gekennzeichnet, dass** man an eine Stelle derselben eine wirksame Dosis einer aktiven Substanz mittels einer Vorrichtung nach einem der Ansprüche 1 bis 6 aufträgt.

10. Verwendung von Hexamethyldisiloxan als inertem Vehikel bei der Herstellung eines kosmetischen oder pharmazeutischen Produkts, welches dazu bestimmt ist, in Form von Aerosot-Partikeln mittels einer Verteilervorrichtung dargereicht zu werden, **dadurch gekennzeichnet, dass** die Zusammensetzung des Produkts in Gewichtsprozentsätzen besteht aus:
- 75 bis 95 % Hexamethyldisiloxan,
- 0,01 bis 15 % therapeutischer oder kosmetischer Substanz
- 0 bis 10 % eines Mittels, das die Thixotropie verbessert, vorzugsweise mindestens 1 %,
- 0 bis 20 % Verdichtungs-Füllstoffen oder kosmetischen Zusätzen.

11. Verwendung von Hexamethyldisiloxan nach Anspruch 10, **dadurch gekennzeichnet, dass** die Zusammensetzung ein Mittel auf der Basis von Siliciumdioxid umfasst, das die Thixotropie des Trägers verbessert.

12. Verwendung von Hexamethyldisiloxan nach einem der Ansprüche 10 und 11, **dadurch gekennzeichnet, dass** die aktive Substanz in Form von Pulver vorliegt.

13. Verwendung von Hexamethyldisiloxan nach einem der Ansprüche 10 bis 12, **dadurch gekennzeichnet, dass** die aktive Substanz ein Antifungusmittel ist, insbesondere auf der Basis von Econazol.

14. Verwendung von Hexamethyldisiloxan nach einem der Ansprüche 10 bis 13, **dadurch gekennzeichnet, dass** die Verteilervorrichtung für Aerosol-Partikel von der Zwei-Taschen-Art ist.

15. Verwendung von Hexamethyldisiloxan nach einem der Ansprüche 10 bis 13, **dadurch gekennzeichnet, dass** die Verteilervorrichtung für Aerosol-Partikel von der Art mit einer Pumpenkammer mit variablem Volumen ist.
